# EUROPÄISCHE PATENTSCHRIFT

(11) **EP 1 836 146 B1**
(45) Veröffentlichungstag und Bekanntmachung des Hinweises auf die Patenterteilung: **05.08.2009**
(21) Anmeldenummer: 06700188.3
(22) Anmeldetag: 04.01.2006
(51) Int. Cl.: C07C 5/327, C07C 7/11, C07C 7/04

(54) **VERFAHREN ZUR HERSTELLUNG VON PROPEN AUS PROPAN**
METHOD FOR THE PRODUCTION OF PROPENE FROM PROPANE
PROCEDE DE PRODUCTION DE PROPENE A PARTIR DE PROPANE

(30) Priorität: 05.01.2005 DE 102005000798; 17.03.2005 DE 102005012291
(43) Veröffentlichungstag der Anmeldung: 26.09.2007
(73) Patentinhaber: BASF SE, 67056 Ludwigshafen (DE)
(72) Erfinder: CRONE, Sven, 67117 Limburgerhof (DE); MACHHAMMER, Otto, 68163 Mannheim (DE); SCHINDLER, Götz-Peter, 68219 Mannheim (DE); BORGMEIER, Frieder, 68163 Mannheim (DE)
(74) Vertreter: Huhn, Michael
(86) Internationale Anmeldenummer: PCT/EP2006/000032
(87) Internationale Veröffentlichungsnummer: WO 2006/072572

(56) Entgegenhaltungen:
- WO-A-02/083615
- US-B1- 6 293 999

## Beschreibung

Die Erfindung betrifft ein Verfahren zur Herstellung von Propen aus Propan.

Propen wird großtechnisch durch Dehydrierung von Propan gewonnen.

In dem als UOP-Oleflex-Prozess bekannten Verfahren zur Dehydrierung von Propan zu Propen wird ein Propan enthaltender Einsatzgasstrom auf 600 - 700 °C vorgeheizt und in einem Wanderbett-Dehydrierreaktor an einem Katalysator, der Platin auf Aluminiumoxid enthält, dehydriert, wobei ein überwiegend Propan, Propen und Wasserstoff enthaltender Produktgasstrom erhalten wird. Daneben sind durch Cracken gebildete leicht siedende Kohlenwasserstoffe (Methan, Ethan, Ethen) sowie geringe Mengen Hochsieder (C₄⁺-Kohlenwasserstoffe) in dem Produktgasstrom enthalten. Das Produktgasgemisch wird abgekühlt und mehrstufig verdichtet. Anschließend werden durch Auskondensieren in einer so genannten "Cold Box" die C₂- und C₃-Kohlenwasserstoffe und die Hochsieder von bei der Dehydrierung gebildetem Wasserstoff und Methan abgetrennt. Das flüssige Kohlenwasserstoff-Kondensat wird anschließend destillativ aufgetrennt, wobei in einer ersten Kolonne die C₂-Kohlenwasserstoffe und verbliebenes Methan abgetrennt und in einer zweiten Destillationskolonne der C₃-Kohlenwasserstoffstrom in eine Propenfraktion mit hohem Reinheitsgrad und eine Propanfraktion, welche auch die C₄⁺-Kohlenwasserstoffe enthält, aufgetrennt wird.

Nachteilig an diesem Verfahren ist der Verlust an C₃-Kohlenwasserstoffen durch das Auskondensieren in der "Cold Box". Wegen der großen Mengen von bei der Dehydrierung gebildetem Wasserstoff und aufgrund des Phasengleichgewichts werden mit dem Wasserstoff/Methan-Abgasstrom auch größere Mengen an C₃-Kohlenwasserstoffen ausgetragen, falls nicht bei sehr tiefen Temperaturen auskondensiert wird. So muss bei Temperaturen von -20 bis -60 °C gearbeitet werden, um den Verlust an C₃-Kohlenwasserstoffen zu begrenzen, die mit dem Wasserstoff/Methan-Abgasstrom ausgetragen werden.

US 6,293,999 offenbart ein Verfahren zur adsorptiven Abtrennung von Propylen aus einem Einsatzstrom enthaltend Propylen und Propan, bei dem der Einsatzstrom über AIPO-14 als Adsorbens geleitet wird und das an dieses adsorbierte Propen anschließend desorbiert wird.

Die Beschreibung erwähnt die Gegenwart weiterer nicht kondensierbarer Bestandteile wie Kohlendioxid, Kohlenmonoxid und Wasserstoff, welche in dem Einsatzstrom neben Propan und Propen vorliegen können, und welche in der nicht adsorbierten Phase verbleiben. Erwähnt werden in Spalte 8, Zeilen 18 bis 42 eine Reihe von Verfahren, in denen die beschriebene Propylen-Abtrennung eingesetzt werden kann. Die Dehydrierung von Propan zu Propen, insbesondere in Gegenwart eines sauerstoffhaltigen Gasstroms unter Bildung eines komplexen Produktgasgemischs, wird nicht erwähnt.

WO 02/083615 betrifft ein Verfahren zur Herstellung von Acrylsäure durch heterogen katalysierte Gasphasenoxidation von Propen mit molekularem Sauerstoff. Auf Seite 20, Zeile 35 bis Seite 21, Zeile 3 wird erwähnt, dass das nach der Abtrennung der Acrylsäure in dem verbleibenden Restgasgemisch enthaltene nicht umgesetzte Propen aus diesem Restgasgemisch durch Inkontaktbringen mit einem hydrophoben organischen Lösungsmittel, das das Propen bevorzugt zu absorbieren vermag, und nachfolgende Desorption und/oder Strippung mit Luft abgetrennt und in das Verfahren zurückgeführt werden kann. Wird Propen im Beisein von Propan oxidiert, werden Propen und Propan gemeinsam abgetrennt und rückgeführt.

Aufgabe der Erfindung ist es, ein verbessertes Verfahren zur Dehydrierung von Propan zu Propen bereitzustellen.

Gelöst wird die Aufgabe durch ein Verfahren zur Herstellung von Propen aus Propan mit den Schritten
A) ein Propan enthaltender Einsatzgasstrom a wird bereitgestellt;
B) der Propan enthaltende Einsatzgasstrom a, gegebenenfalls ein sauerstoffhaltiger Gasstrom und gegebenenfalls Wasserdampf werden in eine Dehydrierzone eingespeist und Propan wird einer Dehydrierung zu Propen unterworfen, wobei ein Produktgasstrom b enthaltend Propan, Propen, Methan, Ethan, Ethen, Kohlenmonoxid, Kohlendioxid, Wasserdampf, gegebenenfalls Wasserstoff und gegebenenfalls Sauerstoff erhalten wird;
C) der Produktgasstrom b wird abgekühlt, gegebenenfalls verdichtet und Wasserdampf wird durch Kondensieren lassen abgetrennt, wobei ein an Wasserdampf abgereicherter Produktgasstrom c erhalten wird;
D) der Produktgasstrom c wird in einer ersten Absorptionszone mit einem selektiv wirkenden inerten Absorptionsmittel, welches selektiv Propen absorbiert, in Kontakt gebracht, wobei ein im Wesentlichen mit Propen beladener Absorptionsmittelstrom d1 und ein Gasstrom d2 enthaltend Propan, Propen, Methan, Ethan, Ethen, Kohlenmonoxid, Kohlendioxid, gegebenenfalls Wasserstoff und gegebenenfalls Sauerstoff erhalten werden;
E) gegebenenfalls wird der Absorptionsmittelstrom d1 in einer ersten Desorptionszone auf einen niedrigeren Druck entspannt, wobei ein im Wesentlichen mit Propen beladener Absorptionsmittelstrom e1 und ein Propen enthaltender Gasstrom e2 erhalten werden, wobei der Gasstrom e2 in die erste Absorptionszone zurückgeführt wird,
F) aus dem im Wesentlichen mit Propen beladenen Absorptionsmittelstrom d1 bzw. e1 wird in mindestens einer (weiteren) Desorptionszone durch Entspannen, Erhitzen und/oder Strippen des Absorptionsmittelstroms d1 bzw. e1 ein Propen enthaltender Gasstrom f1 freigesetzt, wobei das selektiv wirkende Absorptionsmittel zurück gewonnen wird.

In einem ersten Verfahrensteil A wird ein Propan enthaltender Einsatzgasstrom a bereitgestellt. Dieser enthält im Allgemeinen mindestens 80 Vol.-% Propan, vorzugsweise 90 Vol.-% Propan. Daneben enthält der propanhaltige Einsatzgasstrom a im Allgemeinen noch Butane (n-Butan, iso-Butan). Typische Zusammensetzungen des propanhaltigen Einsatzgasstroms sind in DE-A 102 46 119 und DE-A 102 45 585 offenbart. Üblicherweise wird der propanhaltige Einsatzgasstrom a aus liquid petroleum gas (LPG) gewonnen.

In einem Verfahrensteil B wird der Propan enthaltende Einsatzgasstrom in eine Dehydrierzone eingespeist und einer im Allgemeinen katalytischen Dehydrierung unterworfen. Dabei wird Propan in einem Dehydrierreaktor an einem dehydrieraktiven Katalysator teilweise zu Propen dehydriert. Daneben fallen Wasserstoff und in geringen Mengen Methan, Ethan, Ethen und C₄⁺-Kohlenwasserstoffe (n-Butan, iso-Butan, Butene, Butadien) an. Außerdem fallen im Allgemeinen Kohlenstoffoxide (CO, CO₂), insbesondere CO₂ Wasserdampf und gegebenenfalls in geringem Umfang Inertgase im Produktgasgemisch der katalytischen Propan-Dehydrierung an. Der Produktgasstrom der Dehydrierung enthält im Allgemeinen Wasserdampf, der bereits dem Dehydriergasgemisch zugesetzt und/oder - bei Dehydrierung in Gegenwart von Sauerstoff (oxidativ oder nicht-oxidativ) - bei der Dehydrierung gebildet wird. Inertgase (Stickstoff) werden bei Durchführung der Dehydrierung in Gegenwart von Sauerstoff mit dem eingespeisten sauerstoffhaltigen Gasstrom in die Dehydrierzone eingebracht, sofern kein reiner Sauerstoff eingespeist wird. Wird ein sauerstoffhaltiges Gas eingespeist, so liegt dessen Sauerstoff-Gehalt im Allgemeinen bei mindestens 40 Vol.-%, bevorzugt mindestens 80 Vol.-%, besonders bevorzugt mindestens 90 Vol.-%. Insbesondere wird technisch reiner Sauerstoff mit einem Sauerstoffgehalt > 99% angepasst, um einen zu hohen Inertgasanteil im Produktgasgemisch zu vermeiden. Daneben liegt im Produktgasgemisch nicht umgesetztes Propan vor.

Die Propan-Dehydrierung kann grundsätzlich in allen aus dem Stand der Technik bekannten Reaktortypen durchgeführt werden. Eine vergleichsweise ausführliche Beschreibung von erfindungsgemäß geeigneten Reaktortypen enthält auch "Catalytica^{®} Studies Division, Oxidative Dehydrogenation and Alternative Dehydrogenation Processes" (Study Number 4192 OD, 1993,430 Ferguson Drive, Mountain View, California, 94043-5272, USA).

Die Dehydrierung kann als oxidative oder nicht-oxidative Dehydrierung durchgeführt werden. Die Dehydrierung kann isotherm oder adiabat durchgeführt werden. Die Dehydrierung kann katalytisch im Festbett-, Wanderbett- oder Wirbelbettreaktor durchgeführt werden.

Die nicht-oxidative katalytische Propan-Dehydrierung wird bevorzugt autotherm durchgeführt. Dazu wird dem Reaktionsgasgemisch der Propan-Dehydrierung in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird.

Ein Merkmal der nicht-oxidativen Fahrweise gegenüber einer oxidativen Fahrweise ist die zumindest intermediäre Bildung von Wasserstoff, die sich im Vorhandensein von Wasserstoff im Produktgas der Dehydrierung niederschlägt. Bei der oxidativen Dehydrierung findet sich kein freier Wasserstoff im Produktgas der Dehydrierung.

Eine geeignete Reaktorform ist der Festbettrohr- oder Rohrbündelreaktor. Bei diesen befindet sich der Katalysator (Dehydrierungskatalysator und gegebenenfalls spezieller Oxidationskatalysator) als Festbett in einem Reaktionsrohr oder in einem Bündel von Reaktionsrohren. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8 bar, häufig zwischen 1 und 2 bar bei Verwendung einer geringen Wasserdampfverdünnung, aber auch zwischen 3 und 8 bar bei Verwendung einer hohen Wasserdampfverdünnung (entsprechend dem so genannten "steam active reforming process" (STAR-Prozess) oder dem Linde-Verfahren) zur Dehydrierung von Propan oder Butan von Phillips Petroleum Co. Typische Katalysatorbelastungen (GHSV) liegen bei 500 bis 2000 h⁻¹, bezogen auf eingesetzten Kohlenwasserstoff. Die Katalysatorgeometrie kann beispielsweise kugelförmig oder zylindrisch (hohl oder voll) sein.

Die katalytische Propan-Dehydrierung kann auch, entsprechend dem Snamprogetti/Yarsintez-FBD-Prozess, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet.

Der Arbeitsdruck beträgt typischerweise 1 bis 2 bar, die Dehydriertemperatur in der Regel 550 bis 600°C. Die für die Dehydrierung erforderliche Wärme kann dabei in das Reaktionssystem eingebracht werden, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme wird direkt im Reaktorsystem durch Verbrennung von Wasserstoff und/oder Kohlenwasserstoffen in Gegenwart von Sauerstoff erzeugt. Gegebenenfalls kann zusätzlich ein Wasserstoff enthaltender Co-Feed zugemischt werden.

Die katalytische Propan-Dehydrierung kann in einem Hordenreaktor durchgeführt werden. Wird die Dehydrierung unter Einspeisung eines sauerstoffhaltigen Gasstroms autotherm durchgeführt, so wird sie bevorzugt in einem Hordenreaktor durchgeführt. Dieser enthält ein oder mehrere aufeinander folgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer Ausführungsform. In einer weiteren bevorzugten Ausführungsform wird die Dehydrierung in einem Hordenreaktor mit 3 Katalysatorbetten durchgeführt.

Im Allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des Propans benötigte Wärmemenge erzeugt wird. Im Allgemeinen beträgt die insgesamt zugeführte Sauerstoffmenge, bezogen auf die Gesamtmenge des Propans, 0,001 bis 0,8 mol/mol, bevorzugt 0,001 bis 0,6 mol/mol, besonders bevorzugt 0,02 bis 0,5 mol/mol. Sauerstoff kann entweder als reiner Sauerstoff oder als sauerstoffhaltiges Gas, welches Inertgase enthält, eingesetzt werden. Um hohe Propan- und Propen-Verluste bei der Aufarbeitung (siehe unten) zu vermeiden ist es jedoch wesentlich, dass der Sauerstoff-Gehalt des eingesetzten sauerstoffhaltigen Gases hoch ist und mindestens 40 Vol.-%, bevorzugt mindestens 80 Vol.-%, besonders bevorzugt mindestens 90 Vol.-% beträgt. Besonders bevorzugtes sauerstoffhaltiges Gas ist technisch reiner Sauerstoff mit einem O₂-Gehalt von ca. 99 Vol.-%. Der zur Wärmeerzeugung verbrannte Wasserstoff ist der bei der katalytischen Propan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff zu Sauerstoff in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stellen des Reaktors erfolgen.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im Allgemeinen 400 bis 1100 °C, der Druck im letzten Katalysatorbett des Hordenreaktors im Allgemeinen 0,2 bis 15 bar, bevorzugt 1 bis 10 bar, besonders bevorzugt 1 bis 5 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im Allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliziumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Geeignete Katalysatorformkörper-Geometrien sind Stränge, Sterne, Ringe, Sattel, Kugeln, Schäume und Monolithen mit charakteristischen Abmessungen von 1 bis 100 mm.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen Propan-Dehydrierung sind die Katalysatoren gemäß den Beispielen 1,2,3 und 4 der DE-A 199 37 107.

Die autotherme Propan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt. Durch die Verdünnung mit Wasserdampf wird das Gleichgewicht zu den Produkten der Dehydrierung verschoben.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Gegebenenfalls wird der Katalysator nach der Regenerierung mit einem wasserstoffhaltigen Gas reduziert.

Der Produktgasstrom b kann in zwei Teilströme aufgetrennt werden, wobei ein Teilstrom in die autotherme Dehydrierung zurückgeführt wird, entsprechend der in DE-A 102 11 275 und DE-A 100 28 582 beschriebenen Kreisgasfahrweise.

Die Propan-Dehydrierung kann als oxidative Dehydrierung durchgeführt werden. Die oxidative Propan-Dehydrierung kann als homogene oxidative Dehydrierung oder als heterogen katalysierte oxidative Dehydrierung durchgeführt werden.

Gestaltet man im Rahmen des erfindungsgemäßen Verfahrens die Propan-Dehydrierung als eine homogene Oxidehydrierung, so lässt sich diese prinzipiell wie in den Schriften US-A 3,798,283, CN-A 1,105,352, Applied Catalysis, 70 (2), 1991, S. 175 bis 187, Catalysis Today 13, 1992, S. 673 bis 678 und der älteren Anmeldung DE-A 1 96 22 331 beschrieben durchführen.

Die Temperatur der homogenen Oxidehydrierung beträgt im Allgemeinen von 300 bis 700 °C, vorzugsweise von 400 bis 600 °C, besonders bevorzugt von 400 bis 500 °C. Der Druck kann 0,5 bis 100 bar oder 1 bis 50 bar betragen. Häufig wird er bei 1 bis 20 bar, insbesondere bei 1 bis 10 bar liegen.

Die Verweilzeit des Reaktionsgasgemisches unter Oxidehydrierbedingungen liegt üblicherweise bei 0,1 bzw. 0,5 bis 20 sec, vorzugsweise bei 0,1 bzw. 0,5 bis 5 sec. Als Reaktor kann z.B. ein Rohrofen oder ein Rohrbündelreaktor verwendet werden, wie z.B. ein Gegenstromrohrofen mit Rauchgas als Wärmeträger, oder ein Rohrbündelreaktor mit Salzschmelze als Wärmeträger.

Das Propan zu Sauerstoff-Verhältnis im einzusetzenden Ausgangsgemisch kann 0,5 : 1 bis 40: 1 betragen. Vorzugsweise beträgt das Molverhältnis von Propan zu molekularem Sauerstoff im Ausgangsgemisch ≤ 6 : 1, bevorzugt ≤ 5 : 1. In der Regel wird vorgenanntes Verhältnis ≥ 1:1, beispielsweise ≥ 2 : 1 betragen. Das Ausgangsgemisch kann weitere, im Wesentlichen inerte Bestandteile wie H₂O, CO₂, CO, N₂, Edelgase und/oder Propen umfassen. Propen kann in der aus der Raffinerie kommenden C₃-Fraktion enthalten sein. Günstig für eine homogene oxidative Dehydrierung von Propan zu Propen ist es, wenn das Verhältnis der Oberfläche des Reaktionsraumes zum Volumen des Reaktionsraumes möglichst klein ist, da die homogene oxidative Propan-Dehydrierung nach einem radikalischen Mechanismus abläuft und die Reaktionsraumoberfläche in der Regel als Radikalfänger wirkt. Besonders günstige Oberflächenmaterialien sind Aluminiumoxide, Quarzglas, Borosilicate, Edelstahl und Aluminium.

Gestaltet man im Rahmen des erfindungsgemäßen Verfahrens die erste Reaktionsstufe als eine heterogen katalysierte Oxidehydrierung, so lässt sich diese prinzipiell wie in den Schriften US-A 4,788,371, CN-A 1,073,893, Catalysis Letters 23 (1994) 103-106, W. Zhang, Gaodeng Xuexiao Huaxue Xuebao, 14(1993)566, Z. Huang, Shiyou Huagong, 21(1992)592, WO 97/36849, DE-A 1 97 53 817, US-A 3,862,256, US-A 3,887,631, DE-A 195 30 454, US-A 4,341,664, J. of Catalysis 167, 560- 569 (1997), J. of Catalysis 167, 550 - 559 (1997), Topics in Catalysis 3 (1996) 265 - 275, US-A 5,086,032, Catalysis Letters 10 (1991) 181-192, Ind. Eng. Chem. Res. 1996, 35, 14 - 18, US-A 4,255,284, Applied Catalysis A: General, 100 (1993) 111 - 130, J. of Catalysis 148, 56 - 67 (1994), V. Cortés Corberän and S. Vic Bellón (Editors), New Developments in Selective Oxidation II, 1994, Elsevier Science B.V., S. 305 - 313, 3rd World Congress on Oxidation Catalysis R.K. Grasselli, S.T. Oyama, A.M. Gaffney and J.E. Lyons (Editors), 1997, Elsevier Science B.V., S. 375 ff beschrieben durchführen. Insbesondere können alle in den vorgenannten Schriften genannten Oxidehydrierkatalysatoren eingesetzt werden. Das für die vorgenannten Schriften gesagte gilt auch für:
a) Otsuka, K.; Uragami, Y.; Komatsu, T.; Hatano, M. in Natural Gas Conversion, Stud. Surf. Sci. Catal.; Holmen A.; Jens, K.-J.; Kolboe, S., Eds.; Elsevier Science: Amsterdam, 1991; Vol. 61, p 15;
b) Seshan, K.; Swaan, H.M.; Smits, R.H.H.; van Ommen, J.G.; Ross, J.R.H. in New Developments in Selective Oxidation; Stud. Surf. Sci. Catal.; Centi, G.; Trifirò, F., Eds.; Elsevier Science: Amsterdam 1990; Vol. 55, p 505;
c) Smits, R.H.H.; Seshan, K.; Ross, J.R.H. in New Developments in Selective Oxidation by Heterogeneous Catalysis; Stud. Surf. Sci. Catal.; Ruiz, P.; Delmon, B., Eds.; Elsevier Science: Amsterdam, 1992 a; Vol. 72, p 221;
d) Smits, R.H.H.; Seshan, K.; Ross, J.R.H. Proceedings, Symposium on Catalytic Selective Oxidation, Washington DC; American Chemical Society: Washington, DC, 1992 b; 1121;
e) Mazzocchia, C.; Aboumrad, C.; Daigne, C.; Tempesti, E.; Herrmann, J.M.; Thomas, G. Catal. Lett. 1991, 10, 181;
f) Bellusi, G.; Conti, G.; Perathonar, S.; Trifirò, F. Proceedings, Symposium on Catalytic Selective Oxidation, Washington, DC; American Chemical Society: Washington, DC, 1992; p 1242;
g) Ind. Eng. Chem. Res. 1996, 35, 2137 - 2143 und
h) Symposium on Heterogeneons Hydrocarbon Oxidation Presented before the Division of Petroleum Chemistry, Inc. 211 th National Meeting, American Chemical Society New Orleans, LA, March 24 - 29, 1996.

Besonders geeignete Oxidehydrierkatalysatoren sind die Multimetalloxidmassen bzw. - katalysatoren A der DE-A 1 97 53 817, wobei die als bevorzugt genannten Multimetalloxidmassen bzw. -katalysatoren A ganz besonders günstig sind. D.h., als Aktivmassen kommen insbesondere Multimetalloxidmassen der allgemeinen Formel I

M¹ₐMo_{1-b}M²_{b}Oₓ (I),

mit
- M¹ =: Co, Ni, Mg, Zn, Mn und/oder Cu,
- M² =: W, V, Te, Nb, P, Cr, Fe, Sb, Ce, Sn und/oder La,
- a =: 0,5 bis 1,5,
- b =: 0 bis 0,5 sowie
- X =: eine Zahl, die durch die Wertigkeit und Häufigkeit der von Sauerstoff verschie- denen Elemente in I bestimmt wird,
in Betracht.

Weitere als Oxidehydrierungskatalysatoren geeignete Multimetalloxidmassen sind nachstehend genannt:

Geeignete Mo-V-Te/Sb-Nb-O-Multimetalloxidkatalysatoren sind in EP-A 0 318 295, EP-A 0 529 853, EP-A 0 603 838, EP-A 0 608 836, EP-A 0 608 838, EP-A 0 895 809, EP-A 0 962 253, EP-A 1 192 987, DE-A 198 35 247, DE-A 100 51 419 und DE-A 101 19 933 offenbart.

Geeignete Mo-V-Nb-O-Multimetalloxidkatalysatoren sind unter anderem beschrieben in E. M. Thorsteinson, T. P. Wilson, F. G. Young, P. H. Kasei, Journal of Catalysis 52 (1978), Seiten 116 -132 sowie in US 4,250,346 und EP-A 0 294 845.

Geeignete Ni-X-O-Multimetalloxidkatalysatoren, mit X = Ti, Ta, Nb, Co, Hf, W, Y, Zn, Zr, Al, sind in WO 00/48971 beschrieben.

Prinzipiell können geeignete Aktivmassen in einfacher Weise dadurch hergestellt werden, dass man von geeigneten Quellen ihrer Komponenten ein möglichst inniges, vorzugsweise feinteiliges, ihrer Stöchiometrie entsprechend zusammengesetztes Trockengemisch erzeugt und dieses bei Temperaturen von 450 bis 1000 °C calciniert. Die Calcinierung kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, Sauerstoff und NH₃, CO und/oder H₂) erfolgen. Als Quellen für die Komponenten der Multimetalloxidaktivmassen kommen Oxide und/oder solche Verbindungen in Frage, die durch Erhitzen, wenigstens in Anwesenheit von Sauerstoff, in Oxide überführbar sind. Neben den Oxiden kommen als solche Ausgangsverbindungen vor allem Halogenide, Nitrate, Formiate, Oxalate, Citrate, Acetate, Carbonate, Aminkomplexsalze, Ammoniumsalze und/oder Hydroxide in Betracht.

Die Multimetalloxidmassen können für das erfindungsgemäße Verfahren sowohl in Pulverform als auch zu bestimmten Katalysatorgeometrien geformt eingesetzt werden, wobei die Formgebung vor oder nach der abschließenden Calcinierung erfolgen kann. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Geeignete Hohlzylindergeometrien sind z.B. 7 mm x 7 mm x 4 mm oder 5 mm x 3 mm x 2 mm oder 5 mm x 2 mm x 2 mm (jeweils Länge x Außendurchmesser x Innendurchmesser). Selbstverständlich kann der Vollkatalysator auch Kugelgeometrie aufweisen, wobei der Kugeldurchmesser 2 bis 10 mm betragen kann.

Selbstverständlich kann die Formgebung der pulverförmige Aktivmasse oder ihrer pulverförmigen, noch nicht calcinierten, Vorläufermasse auch durch Aufbringen auf vorgeformte inerte Katalysatorträger erfolgen. Die Schichtdicke der auf den Trägerkörper aufgebrachten Pulvermasse wird zweckmäßigerweise im Bereich 50 bis 500 mm, bevorzugt im Bereich 150 bis 250 mm liegend, gewählt. Als Trägermaterialien können dabei übliche poröse oder unporöse Aluminiumoxide, Siliciumdioxid, Thoriumdioxid, Zirkondioxid, Siliciumcarbid oder Silicate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln, Hohlzylinder oder Sättel mit Abmessungen im Bereich von 1 bis 100 mm bevorzugt werden. Geeignet ist die Verwendung von im Wesentlichen unporösen, oberflächenrauhen, kugelförmigen Trägem aus Steatit, deren Durchmesser 1 bis 8 mm, bevorzugt 4 bis 5 mm beträgt.

Die Reaktionstemperatur der heterogen katalysierten Oxidehydrierung des Propans beträgt im Allgemeinen von 300 bis 600 °C, üblicher Weise von 350 bis 500 °C. Der Druck beträgt 0,2 bis 15 bar, bevorzugt 1 bis 10 bar, beispielsweise 1 bis 5 bar. Drücke oberhalb von 1 bar, z.B. 1,5 bis 10 bar, haben sich als besonders vorteilhaft erwiesen. In der Regel erfolgt die heterogen katalysierte Oxidehydrierung des Propans an einem Katalysatorfestbett. Letzteres wird zweckmäßigerweise in den Rohren eines Rohrbündelreaktors aufgeschüttet, wie sie z.B. in der EP-A 700 893 und in der EP-A 700 714 sowie der in diesen Schriften zitierten Literatur beschrieben sind. Die mittlere Verweilzeit des Reaktionsgasgemisches in der Katalysatorschüttung liegt im Normalfall bei 0,5 bis 20 sec. Das Propan zu Sauerstoff Verhältnis im für die heterogen katalysierte Propan-Oxidehydrierung einzusetzenden Reaktionsgasausgangsgemisch kann erfindungsgemäß 0,5 : 1 bis 40 : 1 betragen. Von Vorteil ist es, wenn das Molverhältnis von Propan zu molekularem Sauerstoff im Ausgangsgasgemisch ≤ 6 : 1, vorzugsweise ≤ 5 : 1 beträgt. In der Regel wird vorgenanntes Verhältnis ≥ 1 : 1, beispielsweise 2 : 1 betragen. Das Ausgangsgasgemisch kann weitere, im Wesentlichen inerte Bestandteile wie H₂O, CO₂, CO, N₂, Edelgase und/oder Propen umfassen. Daneben können in gewissem Umfang noch C₁-, C₂- und C₄-Kohlenwasserstoffe enthalten sein.

Der Produktgasstrom b steht beim Verlassen der Dehydrierzone im Allgemeinen unter einem Druck von 0,2 bis 15 bar, vorzugsweise 1 bis 10 bar, besonders bevorzugt 1 bis 5 bar, und weist eine Temperatur im Bereich von 300 bis 700 °C auf.

Bei der Propan-Dehydrierung wird ein Gasgemisch erhalten, welches im Allgemeinen folgende Zusammensetzung aufweist: 10 bis 80 Vol.-% Propan, 5 bis 50 Vol.-% Propen, 0 bis 20 Vol.-% Methan, Ethan, Ethen und C₄⁺-Kohlenwasserstoffe, 0 bis 30 Vol.-% Kohlenstoffoxide, 0 bis 70 Vol.-% Wasserdampf und 0 bis 25 Vol.-% Wasserstoff sowie 0 bis 50 Vol.-% Inertgase.

Bei der bevorzugten autothermen Propan-Dehydrierung wird ein Gasgemisch erhalten, welches im Allgemeinen folgende Zusammensetzung aufweist: 10 bis 80 Vol.-% Propan, 5 bis 50 Vol.-% Propen, 0 bis 20 Vol.-% Methan, Ethan, Ethen und C₄⁺-Kohlenwasserstoffe, 0,1 bis 30 Vol.-% Kohlenstoffoxide, 1 bis 70 Vol.-% Wasserdampf und 0,1 bis 25 Vol.-% Wasserstoff sowie 0 bis 30 Vol.-% Inertgase.

In Verfahrensteil C wird zunächst Wasser aus dem Produktgasstrom b abgetrennt. Die Abtrennung von Wasser wird durch Kondensieren lassen durch Abkühlen und ggf. Verdichten des Produktgasstroms b durchgeführt und kann in einer oder mehreren Abkühlungs- und gegebenenfalls Verdichtungsstufen durchgeführt werden. Im Allgemeinen wird der Produktgasstrom b hierzu auf eine Temperatur im Bereich von 20 bis 80 °C, vorzugsweise 40 bis 65 °C abgekühlt. Zusätzlich kann der Produktgasstrom verdichtet werden, im Allgemeinen auf einen Druck im Bereich von 2 bis 40 bar, bevorzugt 5 bis 20 bar, besonders bevorzugt 10 bis 20 bar.

In einer Ausführungsform des erfindungsgemäßen Verfahrens wird der Produktgasstrom b durch eine Kaskade von Wärmetauschern geleitet und so zunächst auf eine Temperatur im Bereich von 50 bis 200 °C abgekühlt und anschließend in einem Quenchturm mit Wasser auf eine Temperatur von 40 bis 80 °C, beispielsweise 55 °C weiter abgekühlt. Dabei kondensiert der größte Teil des Wasserdampfs aus, aber auch ein Teil der im Produktgasstrom b enthaltenen C₄+-Kohlenwasserstoffe, insbesondere die C₅⁺-Kohlenwasserstoffe. Geeignete Wärmetauscher sind beispielsweise Direktwärmetauscher und Gegenstrom-Wärmetauscher, wie Gas-Gas-Gegenstrom-Wärmetauscher, und Luftkühler.

Es wird ein von Wasserdampf abgereicherter Produktgasstrom c erhalten. Dieser enthält im Allgemeinen noch 0 bis 10 Vol.-% Wasserdampf. Zur praktisch vollständigen Entfernung von Wasser aus dem Produktgasstrom c kann bei Verwendung bestimmter Lösungsmittel in Schritt D) eine Trocknung mittels Molekularsieb oder Membranen vorgesehen werden.

In einem Verfahrensschritt D) wird der Produktgasstroms c in einer ersten Absorptionszone mit einem selektiv wirkenden inerten Absorptionsmittel, welches selektiv Propen absorbiert, in Kontakt gebracht, wobei ein mit C₃-Kohlenwasserstoffen, - im Wesentlichen mit Propen - beladener Absorptionsmittelstrom d1 und ein Gasstrom d2 enthaltend Propan, Methan, Ethan, Ethen, Kohlenmonoxid, Kohlendioxid und Wasserstoff erhalten werden. Auch Propen kann in geringen Mengen in dem Gasstrom d2 enthalten sein.

Vor Durchführung des Verfahrensschritts D) kann aus dem Produktgasstrom c Kohlendioxid durch Gaswäsche abgetrennt werden, wobei ein an Kohlendioxid abgereicherter Produktgasstrom c erhalten wird. Der Kohlendioxid-Gaswäsche kann eine gesonderte Verbrennungsstufe vorgeschaltet werden, in der Kohlenmonoxid selektiv zu Kohlendioxid oxidiert wird.

Zur CO₂-Abtrennung wird im Allgemeinen Natronlauge, Kalilauge oder eine Alkanolamin-Lösung als Waschflüssigkeit eingesetzt, bevorzugt wird eine aktivierte N-Methyldiethanolamin-Lösung eingesetzt. Im Allgemeinen wird vor Durchführung der Gaswäsche der Produktgasstrom c durch ein- oder mehrstufige Verdichtung auf einen Druck im Bereich von 5 bis 25 bar verdichtet.

Es wird ein an Kohlendioxid abgereicherter Produktgasstrom c mit einem CO₂-Gehalt von im Allgemeinen < 100 ppm, vorzugsweise < 10 ppm erhalten.

Die Absorption kann durch einfaches Durchleiten des Stroms c durch das Absorptionsmittel erfolgen. Sie kann aber auch in Kolonnen erfolgen. Dabei kann im Gleichstrom, Gegenstrom oder Kreuzstrom gearbeitet werden. Geeignete Absorptionskolonnen sind z.B. Bodenkolonnen mit Glocken-, Ventil- und/oder Siebböden, Kolonnen mit strukturierten Packungen, z.B. Gewebepackungen oder Blechpackungen mit einer spezifischen Oberfläche von 100 bis 1000 m²/m³ wie Mellapak^{®} 250 Y, und Füllkörperkolonnen, z. B. mit Kugeln, Ringen oder Sätteln aus Metall, Kunststoff oder Keramik als Füllkörpern. Es kommen aber auch Riesel- und Sprühtürme, Graphitblockabsorber, Oberflächenabsorber wie Dickschicht- und Dünnschichtabsorber sowie Blasensäulen mit und ohne Einbauten in Betracht.

Vorzugsweise weist die Absorptionskolonne einen Absorptionsteil und einen Rektifikationsteil auf. Das Absorptionsmittel wird dabei im Allgemeinen am Kolonnenkopf aufgegeben, der Strom c wird im Allgemeinen in der Mitte oder der oberen Hälfte der Kolonne eingespeist. Zur Erhöhung der Propen-Anreicherung im Lösungsmittel nach Art einer Rektifikation kann dann in den Kolonnensumpf Wärme eingetragen werden. Alternativ kann in den Kolonnensumpf ein Strippgasstrom eingespeist werden, beispielsweise aus Stickstoff, Luft, Wasserdampf oder Propen, vorzugsweise aus Propen. Ein Teil des Kopfproduktes kann kondensiert und als Rücklauf wieder auf den Kolonnenkopf gegeben werden, um Lösungsmittelverluste zu begrenzen.

Geeignete selektiv wirkende Absorptionsmittel, die selektiv Propen absorbieren, sind beispielsweise N-Methylpyrrolidon (NMP), NMP/Wasser-Gemische mit bis zu 20 Gew.-% Wasser, m-Kresol, Essigsäure, Methylpyrazin, Dibrommethan, Dimethylformamid (DMF), Propylencarbonat, N-Formylmorpholin, Ethylencarbonat, Formamid, Malonodinitril, gamma-Butyrolacton, Nitrobenzol, Dimethylsulfoxid (DMSO), Sulfolan, Pyrrol, Milchsäure, Acrylsäure, 2-Chlorpropionsäure, Triallyltrimellitat, Tris(2-ethylhexyl)trimellitat, Dimethylphthalat, Bernsteinsäuredimethylester, 3-Chlorpropionsäure, Morpholin, Acetonitril, 1-Butyl-3-methylimidazoliniumoctylsulfat, Ethylmethylimidazoliniumtosylat, Dimethylanilin, Adiponitril und Ameisensäure.

Bevorzugte selektiv absorbierende Absorptionsmittel sind NMP, NMP/Wasser-Gemische mit bis zu 20 Gew.-% Wasser, Acetonitril sowie Gemische aus Acetonitril, organischen Lösungsmitteln und/oder Wasser mit einem Acetonitrilgehalt von ≥ 50 Gew.-% sowie Dimethylanilin.

Der Absorptionsschritt D) wird im Allgemeinen bei einem Druck von 2 bis 40 bar, vorzugsweise von 5 bis 20 bar, besonders bevorzugt von 10 bis 20 bar durchgeführt. Neben Propen wird in gewissem Umfang auch Propan durch das selektiv wirkende Absorptionsmittel mit absorbiert. Daneben können auch noch in geringen Mengen Ethen und Butene mit absorbiert werden.

In einem optionalen Schritt E) wird der Absorptionsmittelstrom d1 in einer ersten Desorptionszone auf einen niedrigeren Druck entspannt, wobei ein im Wesentlichen mit Propen beladener Absorptionsmittelstrom e1 und ein überwiegend Propen enthaltender Gasstrom e2, der noch geringe Mengen Propan enthält, erhalten werden, wobei der Gasstrom e2 in die erste Absorptionszone, vorzugsweise als Strippgas in den Rektifikationsteil der Absorptionskolonne, zurückgeführt wird.

Hierzu wird der Absorptionsmittelstrom d1 von einem Druck, der dem Druck der Absorptionsstufe D) entspricht, auf einen Druck von im Allgemeinen 1 bis 20 bar, bevorzugt 1 bis 10 bar entspannt. Die Entspannung kann mehrstufig, im Allgemeinen bis zu 5-stufig, beispielsweise 2-stufig durchgeführt werden. Zusätzlich kann der beladene Absorptionsmittelstrom noch erwärmt werden.

Es wird ein Propen enthaltender Gasstrom e2 erhalten, der im Allgemeinen 0 bis 5 Vol.-% Propan, 50 bis 99,9 Vol.-% Propen und 0 bis 15 Vol.-% weitere Gasbestandteile wie Wasserdampf, Ethylen und Kohlenstoffoxide sowie 0 bis 50 Vol.-% Lösungsmittel enthält. Dieser wird in die Absorptionszone zurückgeführt. Vorzugsweise wird der rückgeführte Gasstrom e2 im unteren Teil der Absorptionskolonne, beispielsweise auf der Höhe des 1. - 10. theoretischen Bodens, zugegeben. Durch den rückgeführten Propen-Strom wird in dem Absorptionsmittel gelöstes Propan herausgestrippt und so der Propen-Anreicherungsgrad in dem Absorptionsmittel erhöht.

In einem Schritt F) wird aus dem im Wesentlichen noch mit Propen beladenen Absorptionsmittelstrom d1 bzw. e1 in mindestens einer (zweiten) Desorptionszone durch Entspannen, Erhitzen und/oder Strippen des Absorptionsmittelstroms d1 bzw. e1 ein Propen enthaltender Gasstrom f1 freigesetzt, wobei das selektiv wirkende Absorptionsmittel zurück gewonnen wird. Gegebenenfalls wird ein Teil dieses Absorptionsmittelstroms, der C₄⁺-Kohlenwasserstoffe enthalten kann, ausgeschleust, aufgearbeitet und zurückgeführt, oder verworfen.

Zur Desorption der in dem Absorptionsmittel gelösten Gase wird dieses erhitzt und/oder auf einen niedrigeren Druck entspannt. Alternativ dazu kann die Desorption auch durch Strippung, üblicher Weise mit Wasserdampf, oder in einer Kombination von Entspannung, Erhitzen und Strippung in einem oder mehreren Verfahrensschritten erfolgen.

Der durch Desorption freigesetzte, Propen enthaltender Gasstrom f1 enthält im Allgemeinen, bezogen auf den Kohlenwasserstoffgehalt, mindestens 98 Vol.-% Propen, vorzugsweise mindestens 99 Vol.-% Propen, besonders bevorzugt mindestens 99,5 Vol.-% Propen. Daneben kann er 0 bis 2 Vol.-% Propan enthalten sowie geringe Mengen leichtsiedender Kohlenwasserstoffe wie Methan und Ethen, im Allgemeinen aber nicht mehr als 0,5 Vol.-%, vorzugsweise nicht mehr als 0,2 Vol.-%. Wird zur Desorption mit Wasserdampf gestrippt, so enthält der Gasstrom f1 noch Wasserdampf, im Allgemeinen in Mengen bis zu 50 Vol.-%, bezogen auf den gesamten Gasstrom.

Wird zur Desorption von Propen im Verfahrensteil F mit Wasserdampf gestrippt, so wird im Allgemeinen anschließend der Wasserdampf aus dem Gasstrom f1 wieder abgetrennt. Diese Abtrennung kann durch Kondensieren lassen durch Abkühlen und ggf. Verdichten des Gasstroms f1 bewirkt werden. Die Abtrennung kann in einer oder mehreren Abkühlungs- und gegebenenfalls Verdichtungsstufen durchgeführt werden.

Im Allgemeinen wird der Gasstrom f1 hierzu auf eine Temperatur im Bereich von 0 bis 80 °C, vorzugsweise 10 bis 65 °C abgekühlt. Zusätzlich kann der Produktgasstrom verdichtet werden, beispielsweise auf einen Druck im Bereich von 2 bis 50 bar. Zur praktisch vollständigen Entfernung von Wasser aus dem Gasstrom f1 kann eine Trocknung mittels Molekularsieb vorgesehen werden. Die Trocknung kann auch durch Adsorption, Membrantrennung, Rektifikation oder weiteren aus dem Stand der Technik bekannten Trocknungsverfahren erfolgen.

Um einen besonders hohen Propen-Gehalt des Gasstroms f1 zu erzielen, wird vorzugsweise ein Teil des in Schritt F) erhaltenen, Propen enthaltenden Gasstroms f1 in die Absorptionszone zurückgeführt. Der Anteil des rückgeführten Gasstroms beträgt im Allgemeinen 0 bis 25 %, vorzugsweise 0 bis 10 % des Gasstroms f1.

Im Allgemeinen wird zumindest ein Teil des in dem Gasstrom d2 enthaltenen Propan in die Dehydrierzone zurückgeführt.

In einer Ausführungsform des erfindungsgemäßen Verfahrens wird der Propan enthaltende Gasstrom d2 zumindest teilweise direkt in die Dehydrierzone zurückgeführt, wobei im Allgemeinen zur Ausschleusung von Inertgasen, Wasserstoff und Kohlenstoffoxiden ein Teilstrom (Purge-Gasstrom) von dem Gasstrom d2 abgetrennt wird. Der Purge-Gasstrom kann verbrannt werden. Es kann aber auch ein Teilstrom des Gasstroms d2 direkt in die Dehydrierzone zurückgeführt und aus einem weiteren Teilstrom durch Absorption und Desorption Propan abgetrennt und in die Dehydrierzone zurückgeführt werden.

In einer weiteren, bevorzugten Ausführungsform des erfindungsgemäßen Verfahrens wird zumindest ein Teil des in Schritt D) erhaltenen, Propan enthaltenden Gasstroms d2 in einem weiteren Schritt G) mit einem hoch siedenden Absorptionsmittel in Kontakt gebracht und werden die in dem Absorptionsmittel gelösten Gase nachfolgend desorbiert, wobei ein im Wesentlichen aus Propan bestehenden Rückführstrom g1 und ein Abgasstrom g2 enthaltend Methan, Ethan, Ethen, Kohlenmonoxid, Kohlendioxid und Wasserstoff erhalten wird. Der im Wesentlichen aus Propan bestehende Rückführstrom wird in die erste Dehydrierzone zurückgeführt.

Dazu wird in einer Absorptionsstufe der Gasstrom d2 mit einem inerten Absorptionsmittel in Kontakt gebracht, wobei Propan und auch geringe Mengen der C₂-Kohlenwasserstoffe in dem inerten Absorptionsmittel absorbiert werden und ein mit Propan beladenes Absorptionsmittel und ein die übrigen Gasbestandteile enthaltendes Abgas erhalten werden. Im Wesentlichen sind dies Kohlenstoffoxide, Wasserstoff, Inertgase sowie C₂-Kohlenwasserstoffe und Methan. In einer Desorptionsstufe wird Propan aus dem Absorptionsmittel wieder freigesetzt.

In der Absorptionsstufe eingesetzte inerte Absorptionsmittel sind im Allgemeinen hochsiedende unpolare Lösungsmittel, in denen das abzutrennende Propan eine deutlich höhere Löslichkeit als die übrigen Gasbestandteile aufweist. Die Absorption kann durch einfaches Durchleiten des Stroms d2 durch das Absorptionsmittel erfolgen. Sie kann aber auch in Kolonnen oder in Rotationsabsorbem erfolgen. Dabei kann im Gleichstrom, Gegenstrom oder Kreuzstrom gearbeitet werden. Geeignete Absorptionskolonnen sind z.B. Bodenkolonnen mit Glocken-, Zentrifugal- und/oder Siebböden, Kolonnen mit strukturierten Packungen, z.B. Gewebepackungen oder Blechpackungen mit einer spezifischen Oberfläche von 100 bis 1000 m²/m³ wie Mellapak^{®} 250 Y, und Füllkörperkolonnen. Es kommen aber auch Riesel- und Sprühtürme, Graphitblockabsorber, Oberflächenabsorber wie Dickschicht- und Dünnschichtabsorber sowie Rotationskolonnen, Tellerwäscher, Kreuzschleierwäscher, Rotationswäscher sowie Blasensäulen mit und ohne Einbauten in Betracht.

Geeignete Absorptionsmittel sind vergleichsweise unpolare organische Lösungsmittel, beispielsweise aliphatische C₄-C₁₈-Alkene, Naphtha oder aromatische Kohlenwasserstoffe wie die Mittelölfraktionen aus der Paraffmdestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.-%. Geeignete Absorptionsmittel sind ferner Butane, Pentane, Hexane, Heptane, Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

Zur Desorption von Propan wird das beladene Absorptionsmittel erhitzt und/oder auf einen niedrigeren Druck entspannt. Alternativ dazu kann die Desorption auch durch Strippung, üblicher Weise mit Wasserdampf oder einem sauerstoffhaltigen Gas, oder in einer Kombination von Entspannung, Erhitzen und Strippung in einem oder mehreren Verfahrensschritten erfolgen. Beispielsweise kann die Desorption zweistufig durchgeführt werden, wobei die zweite Desorptionsstufe bei einem niedrigeren Druck als die erste Desorptionsstufe durchgeführt wird und das Desorptionsgas der ersten Stufe in die Absorptionsstufe zurückgeführt wird. Das in der Desorptionsstufe regenerierte Absorptionsmittel wird in die Absorptionsstufe zurückgeführt.

In einer Verfahrensvariante wird der Desorptionsschritt durch Entspannung und/oder Erhitzen des beladenen Desorptionsmittels durchgeführt. In einer weiteren Verfahrensvariante wird zusätzlich mit Wasserdampf gestrippt. In einer weiteren Verfahrensvariante wird zusätzlich mit einem sauerstoffhaltigen Gas gestrippt. Die Menge des eingesetzten Strippgases kann dem Sauerstoffbedarf der autothermen Dehydrierung entsprechen.

Alternativ kann im Verfahrensschritt G) Kohlendioxid durch Gaswäsche aus dem Gasstrom d2 oder einem Teilstrom desselben abgetrennt werden, wobei ein an Kohlendioxid abgereicherter Rückführstrom g1 erhalten wird. Der Kohlendioxid-Gaswäsche kann eine gesonderte Verbrennungsstufe vorgeschaltet werden, in der Kohlenmonoxid selektiv zu Kohlendioxid oxidiert wird.

Zur CO₂-Abtrennung wird im Allgemeinen Natronlauge, Kalilauge oder eine Alkanolamin-Lösung als Waschflüssigkeit eingesetzt, bevorzugt wird eine aktivierte N-Methyldiethanolamin-Lösung eingesetzt. Im Allgemeinen wird vor Durchführung der Gaswäsche der Produktgasstrom c durch ein- oder mehrstufige Verdichtung auf einen Druck im Bereich von 5 bis 25 bar verdichtet. Es kann ein an Kohlendioxid abgereicherter Rückführstrom g1 mit einem CO₂-Gehalt von im Allgemeinen < 100 ppm, vorzugsweise < 10 ppm erhalten werden.

Wasserstoff kann gegebenenfalls durch Membrantrennung oder Druckwechselabsorption aus dem Gasstrom d2 abgetrennt werden.

Zur Abtrennung des im Abgasstrom enthaltenen Wasserstoffs kann dieser, gegebenenfalls nach erfolgter Kühlung, beispielsweise in einem indirekten Wärmetauscher, über eine in der Regel als Rohr ausgebildete Membran geleitet werden, die lediglich für molekularen Wasserstoff durchlässig ist. Der so abgetrennte molekulare Wasserstoff kann bei Bedarf zumindest teilweise in der Dehydrierung eingesetzt oder aber einer sonstigen Verwertung zugeführt werden, beispielsweise zur Erzeugung elektrischer Energie in Brennstoffzellen eingesetzt werden. Alternativ kann der Abgasstrom verbrannt werden.

Die Erfindung wird durch das nachstehende Beispiel näher erläutert.

### Beispiel

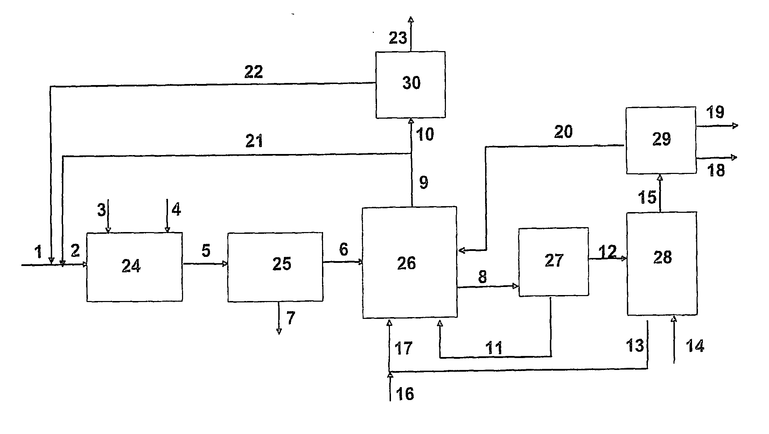

Die in der Figur dargestellte Variante des erfindungsgemäßen Verfahrens wurde rechnerisch simuliert. Dabei wurden die nachfolgenden Verfahrensparameter angenommen.

Es wird eine Kapazität der Anlage von 320 kt/a Propylen bei 8000 h Laufzeit angenommen.

Frisch-Propan enthält typischer Weise neben 98 Gew.-% Propan etwa 2 Gew.-% Butan. Der Butangehalt könnte in einer C3/C4-Trennkolonne mit 40 theoretischen Stufen bei einem Betriebsdruck von 10 bar und einem Rücklaufverhältnis von 0,41 auf 0,01 Gew.-% abgereichert werden. Für den Frisch-Propanstrom 1 wird nachfolgend ein Propangehalt von 100 % angenommen.

Der Frisch-Propanstrom 1 wird mit den Rückführströmen 21 und 22 zum Propan-Einsatzstrom 2 vereinigt. Der Propanstrom 2 wird auf 400 °C vorgeheizt, tritt unter einem Druck von ca. 3 bar in die Dehydrierzone 24 ein und wird einer autothermen Dehydrierung unterworfen. In die Dehydrierzone 24 werden weiterhin ein Strom aus reinem Sauerstoff 3 und ein Wasserdampfstrom 4 eingespeist. Der Umsatz der Dehydrierung beträgt, bezogen auf Propan, 35,3 %, die Selektivität der Propen-Bildung beträgt 95,5%. Es werden daneben 0,8 % Crack-Produkte (Ethan und Ethen) und 3,7 % Kohlenstoffoxide durch Totalverbrennung gebildet. Die Wasserkonzentration im Austrittsgas 5 der Dehydrierzone beträgt 21 Gew.-%, der Restsauerstoffgehalt im Austrittsgas beträgt 0 Gew.-%, die Austrittstemperatur des Produktgasgemischs beträgt 595 °C.

Das Austrittsgas wird bei 2,5 bar auf 55 °C abgekühlt und Wasser bis zum Sättigungsdampfdruck auskondensiert. Anschließend wird das Produktgasgemisch in einem 2-stufigen Verdichter 25 mit Zwischenkühlung 2-stufig verdichtet. In der ersten Verdichterstufe wird von 2,5 bar auf 6 bar und in der zweiten Verdichterstufe von 5,9 bar auf 15,3 bar verdichtet. Nach der ersten Verdichterstufe wird das Gasgemisch auf 55 °C und nach der zweiten Verdichterstufe auf 30 °C abgekühlt. Dabei fällt ein im Wesentlichen aus Wasser bestehender Kondensatstrom 7 an. Der verdichtete und abgekühlte Gasstrom 6 wird in der Absorptionskolonne 26 mit einem Wasser/NMP-Gemisch 17 als Absorptionsmittel bei einem Druck von 15 bar in Kontakt gebracht. Das Absorptionsmittel 17 wird am Kolonnenkopf aufgegeben. Der mit Propen beladene Sumpfabzugsstrom 8 der Absorptionskolonne 26 enthält nur noch geringe Mengen an Propan, so dass eine Propan/Propen-Trennung im weiteren Verlauf der Aufarbeitung unterbleiben kann. Der propanhaltige Kopfabzugsstrom 9 der Absorptionskolonne 26 wird zum Teil als Strom 21 in die Dehydrierzone 24 zurückgeführt. Der verbleibende Teilstrom 10 wird in der Absorptions-/Desorptionseinheit 30 mit Tetradecan (TDK) als Absorptionsmittel in Kontakt gebracht. Der verbleibende Restgasstrom 23 enthält überwiegend Wasserstoff und Kohlenstoffoxide. Durch Desorption wird ein überwiegend Propan enthaltender Gasstrom 22 erhalten, der in die Dehydrierzone 24 zurückgeführt wird. Der Sumpfabzugsstrom 8 aus mit Propen beladenem Absorptionsmittel wird in einer ersten Desorptionsstufe 27 auf einen Druck von 6 bar entspannt. Dabei wird ein überwiegend Propen enthaltender Gasstrom 11 freigesetzt, der in die Absorptionskolonne 26 zurückgeführt wird. Das mit Propen beladene Absorptionsmittel wird als Strom 12 einer Desorptionskolonne 28 zugeführt. In der Desorptionskolonne 28 wird durch Entspannen auf einen Druck von 1,2 bar, Erwärmen des Sumpfes und Strippen mit 16 bar Hochdruckdampf 14 Propen desorbiert, wobei ein Strom 13 aus regeneriertem Absorptionsmittel und ein Strom 15 aus Propen und Wasserdampf erhalten wird. Das regenerierte Absorptionsmittel 13 wird um frisches Absorptionsmittel 16 ergänzt und in die Absorptionskolonne 26 zurückgeführt. Der über den Kolonnenkopf abgezogene Strom 15 wird mehrstufig auf 15 bar verdichtet und dabei stufenweise auf 40 °C abgekühlt. Dabei kondensiert Wasser aus, das als Abwasserstrom 18 aus dem Verfahren ausgeschleust wird, und es wird ein praktisch wasserfreier Reinpropen-Strom 19 erhalten. Ein von Wasserdampf abgereicherter Reinpropen-Strom 20 wird in die Absorptionskolonne zurückgeführt.

Die Zusammensetzung der Ströme in Massenanteilen gibt die nachstehende Tabelle wieder.

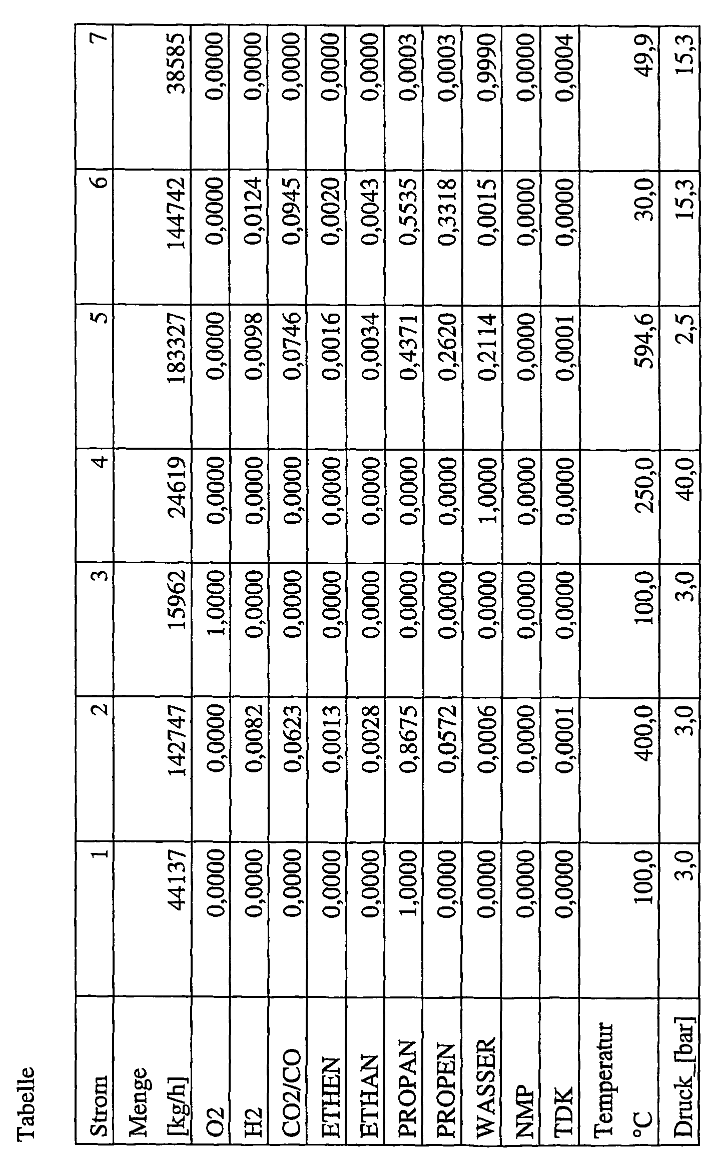

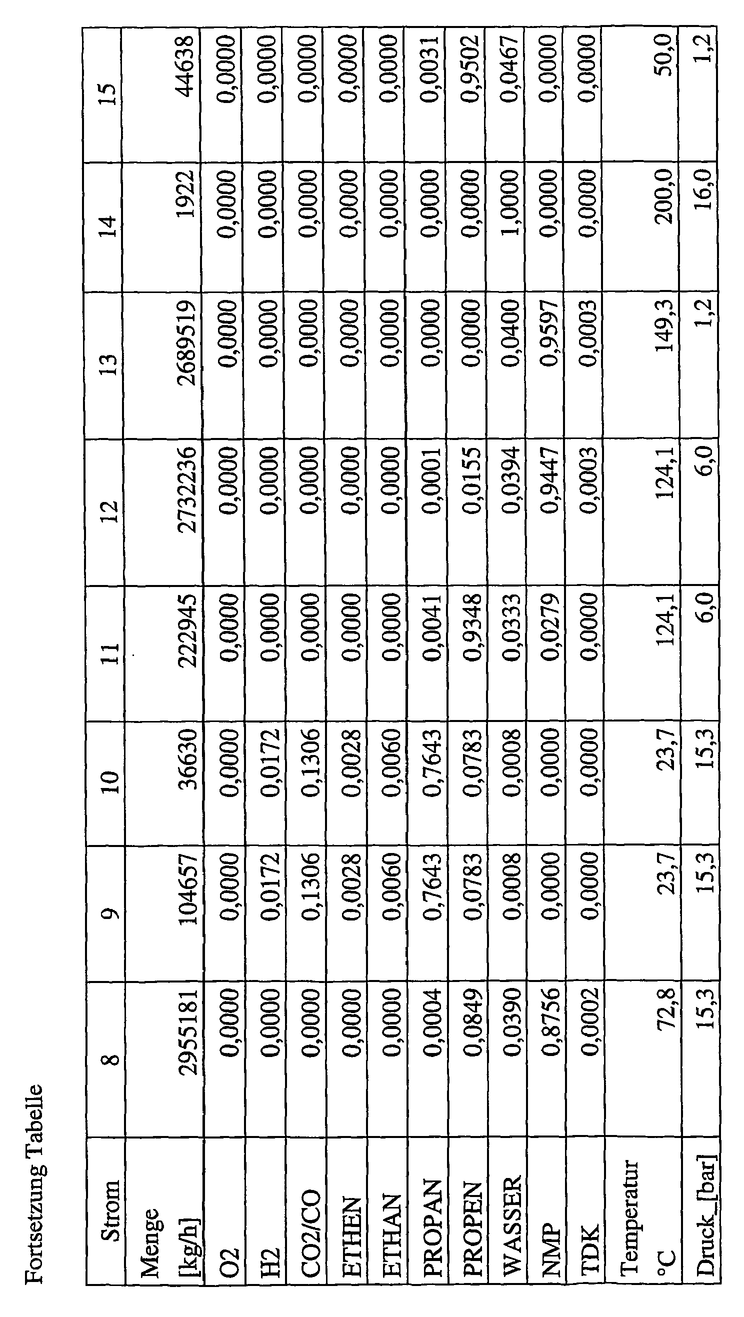

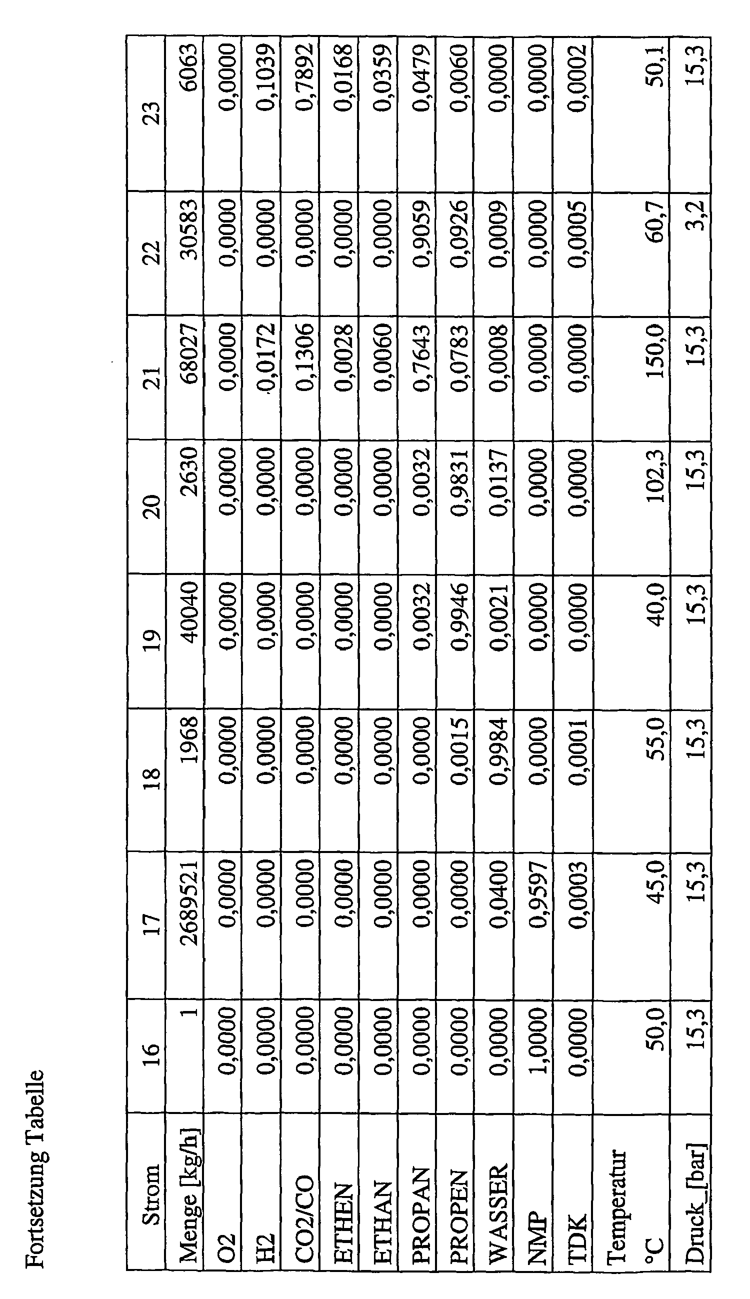

## Patentansprüche

1. Verfahren zur Herstellung von Propen aus Propan mit den Schritten
A) ein Propan enthaltender Einsatzgasstrom a wird bereitgestellt;
B) der Propan enthaltende Einsatzgasstrom a, gegebenenfalls Wasserdampf und gegebenenfalls ein sauerstoffhaltiger Gasstrom werden in eine Dehydrierzone eingespeist und Propan wird einer Dehydrierung zu Propen unterworfen, wobei ein Produktgasstrom b enthaltend Propan, Propen, Methan, Ethan, Ethen, Kohlenmonoxid, Kohlendioxid, Wasserdampf, gegebenenfalls Wasserstoff und gegebenenfalls Sauerstoff erhalten wird;
C) der Produktgasstrom b wird abgekühlt, gegebenenfalls verdichtet und es wird Wasserdampf durch Kondensieren lassen abgetrennt, wobei ein an Wasserdampf abgereicherter Produktgasstrom c erhalten wird;
D) der Produktgasstrom c wird in einer ersten Absorptionszone mit einem selektiv wirkenden inerten Absorptionsmittel, welches selektiv Propen absorbiert, in Kontakt gebracht, wobei ein im Wesentlichen mit Propen beladener Absorptionsmittelstrom d1 und ein Gasstrom d2 enthaltend Propan, Propen, Methan, Ethan, Ethen, Kohlenmonoxid, Kohlendioxid, gegebenenfalls Wasserstoff und gegebenenfalls Sauerstoff erhalten werden;
E) gegebenenfalls wird der Absorptionsmittelstrom d1 in einer ersten Desorptionszone auf einen niedrigeren Druck entspannt, wobei ein im Wesentlichen mit Propen beladener Absorptionsmittelstrom e1 und ein Propen enthaltender Gasstrom e2 erhalten werden, wobei der Gasstrom e2 in die erste Absorptionszone zurückgeführt wird,
F) aus dem im Wesentlichen mit Propen beladenen Absorptionsmittelstrom d1 bzw. e1 wird in mindestens einer zweiten Desorptionszone durch Entspannen, Erhitzen und/oder Strippen des Absorptionsmittelstroms d1 bzw. e1 ein Propen enthaltender Gasstrom f1 freigesetzt, wobei das selektiv wirkende Absorptionsmittel zurück gewonnen wird.

2. Verfahren nach Anspruch 1, **dadurch gekennzeichnet, dass** die Dehydrierung in Schritt B) als oxidative oder nicht-oxidative Dehydrierung durchgeführt wird.

3. Verfahren nach Anspruch 1, **dadurch gekennzeichnet, dass** die Dehydrierung in Schritt B) adiabat oder isotherm durchgeführt wird.

4. Verfahren nach Anspruch 1, **dadurch gekennzeichnet, dass** die Deyhdrierung in Schritt B) in einem Festbettreaktor, Wanderbettreaktor oder Wirbelbettreaktor durchgeführt wird.

5. Verfahren nach Anspruch 1, **dadurch gekennzeichnet, dass** in Schritt B) ein sauerstoffhaltiger Gasstrom eingespeist wird, wobei der sauerstoffhaltige Gasstrom mindestens 90 Vol.-% Sauerstoff enthält.

6. Verfahren nach Anspruch 5, **dadurch gekennzeichnet, dass** die Dehydrierung als autotherme Dehydrierung durchgeführt wird.

7. Verfahren nach einem der Ansprüche 1 bis 6, **dadurch gekennzeichnet, dass** ein Teil des in Schritt F) erhaltenen, Propen enthaltenden Gasstroms f1 in die Absorptionszone zurückgeführt wird.

8. Verfahren nach einem der Ansprüche 1 bis 7, **dadurch gekennzeichnet, dass** das in Schritt D) eingesetzte selektiv wirkende Absorptionsmittel ausgewählt ist aus der Gruppe bestehend aus NMP, NMP/Wasser-Gemischen mit bis zu 20 Gew.-% Wasser, m-Kresol, Essigsäure, Methylpyrazin, Dibrommethan, DMF, Propylencarbonat, N-Formylmorpholin, Ethylencarbonat, Formamid, Malonodinitril, gamma-Butyrolacton, Nitrobenzol, DMSO, Sulfolan, Pyrrol, Milchsäure, Acrylsäure, 2-Chlorpropionsäure, Triallyltrimellitat, Tris(2-ethylhexyl)trimellitat, Dimethylphthalat, Bemsteinsäuredimethylester, 3-Chlorpropionsäure, Morpholin, Acetonitril, 1-Butyl-3-methylimidazoliniumoctylsulfat, Ethylmethylimidazoliniumtosylat, Adiponitril, Dimethylanilin und Ameisensäure.

9. Verfahren nach einem der Ansprüche 1 bis 8, **dadurch gekennzeichnet, dass** in Schritt D) die Absorptionszone als Absorptionskolonne mit einem Absorptionsteil und einem Rektifikationsteil ausgestaltet ist, und in den Kolonnensumpf Wärme und/oder ein Strippgas eingespeist wird.

10. Verfahren nach Anspruch 9, **dadurch gekennzeichnet, dass** in den Kolonnensumpf der Absorptionskolonne als Strippgas ein Propen enthaltender Gasstrom eingespeist wird, welcher im Desorptionsschritt E) gewonnen wird.

11. Verfahren nach einem der Ansprüche 1 bis 10, **dadurch gekennzeichnet, dass** in Schritt F) mit Wasserdampf gestrippt wird.

12. Verfahren nach Anspruch 11, **dadurch gekennzeichnet, dass** aus dem in Schritt F) erhaltenen, Propen und Wasserdampf enthaltenden Gasstrom f1 durch ein- oder mehrstufige Abkühlung und Kompression Wasserdampf als Wasser auskondensiert und abgetrennt wird oder Wasserdampf durch Adsorption, Rektifikation und/oder Membrantrennung abgetrennt wird.

13. Verfahren nach einem der Ansprüche 1 bis 12, **dadurch gekennzeichnet, dass** der in Schritt D) erhaltene Propan enthaltende Abgasstrom d2 zumindest teilweise in die Dehydrierzone zurückgeführt wird.

14. Verfahren nach einem der Ansprüche 1 bis 13, **dadurch gekennzeichnet, dass** zumindest ein Teil des in Schritt D) erhaltenen Propan enthaltenden Gasstroms d2 in einem weiteren Schritt G) mit einem hoch siedenden Absorptionsmittel in Kontakt gebracht und die in dem Absorptionsmittel gelösten Gase nachfolgend desorbiert werden, wobei ein im Wesentlichen aus Propan bestehender Rückführstrom g1 und ein Abgasstrom g2 enthaltend Methan, Ethan, Ethen, Kohlenmonoxid, Kohlendioxid und Wasserstoff erhalten wird, und der im Wesentlichen aus Propan bestehende Rückführstrom g1 in die Dehydrierzone zurückgeführt wird.

15. Verfahren nach Anspruch 14, **dadurch gekennzeichnet, dass** das in Schritt G) eingesetzte hoch siedende Absorptionsmittel ausgewählt ist aus der Gruppe bestehend aus C₄-C₁₈-Alkanen, Naphtha und der Mittelölfraktion aus der Paraffindestillation.

16. Verfahren nach Anspruch 14 oder 15, **dadurch gekennzeichnet, dass** in Schritt G) zur Desorption der in dem Absorptionsmittel gelösten Gase mit Wasserdampf gestrippt wird.

17. Verfahren nach einem der Ansprüche 1 bis 13, **dadurch gekennzeichnet, dass** zumindest aus einem Teilstrom des aus dem in Schritt D) erhaltenen Propan enthaltenden Gasstroms d2 in einem weiteren Schritt G) Kohlendioxid durch Gaswäsche abgetrennt wird, wobei ein kohlendioxid armer Rückführstrom g1 erhalten wird, der in die Dehydrierzone zurückgeführt wird.

## Claims

1. A process for preparing propene from propane, comprising the steps:
A) a feed gas stream a comprising propane is provided;
B) the feed gas stream a comprising propane, optionally steam and optionally an oxygenous gas stream are fed into a dehydrogenation zone and propane is subjected to a dehydrogenation to propene to obtain a product gas stream b comprising propane, propene, methane, ethane, ethene, carbon monoxide, carbon dioxide, steam, with or without hydrogen and with or without oxygen;
C) product gas stream b is cooled and optionally compressed, and steam is removed by condensation to obtain a steam-depleted product gas stream c;
D) product gas stream c is contacted in a first absorption zone with a selective, inert absorbent which selectively absorbs propene to obtain an absorbent stream d1 laden substantially with propene and a gas stream d2 comprising propane, propene, methane, ethane, ethene, carbon monoxide and carbon dioxide, with or without hydrogen and with or without oxygen;
E) if appropriate, the absorbent stream d1 is decompressed to a lower pressure in a first desorption zone to obtain an absorbent stream e1 laden substantially with propene and a gas stream e2 comprising propene, and gas stream e2 is recycled into the first absorption zone,
F) from the absorbent stream d1 or e1 laden substantially with propene, in at least one second desorption zone, by decompression, heating and/or stripping the absorbent stream d1 or e1, a gas stream f1 comprising propene is released and the selective absorbent is recovered.

2. The process according to claim 1, wherein the dehydrogenation in step B) is performed as an oxidative or nonoxidative dehydrogenation.

3. The process according to claim 1, wherein the dehydrogenation in step B) is performed adiabatically or isothermally.

4. The process according to claim 1, wherein the dehydrogenation in step B) is performed in a fixed bed reactor, moving bed reactor or fluidized bed reactor.

5. The process according to claim 1, wherein an oxygen-containing gas stream is fed in in step B), said oxygen-containing gas stream comprising at least 90% by volume of oxygen.

6. The process according to claim 5, wherein the dehydrogenation is performed as an autothermal dehydrogenation.

7. The process according to any of claims 1 to 6, wherein a portion of the gas stream f1 which comprises propene and is obtained in step F) is recycled into the absorption zone.

8. The process according to any of claims 1 to 7, wherein the selective absorbent used in step D) is selected from the group consisting of NMP, NMP/water mixtures comprising up to 20% by weight of water, m-cresol, acetic acid, methylpyrazine, dibromomethane, DMF, propylene carbonate, N-formylmorpholine, ethylene carbonate, formamide, malononitrile, gamma-butyrolactone, nitrobenzene, DMSO, sulfolane, pyrrole, lactic acid, acrylic acid, 2-chloropropionic acid, triallyl trimellitate, tris(2-ethylhexyl)trimellitate, dimethyl phthalate, dimethyl succinate, 3-chloropropionic acid, morpholine, acetonitrile, 1-butyl-3-methylimidazolinium octylsulfate, ethylmethylimidazolinium tosylate, adiponitrile, dimethylaniline and formic acid.

9. The process according to any of claims 1 to 8, wherein the adsorption zone in step D) is configured as an absorption column with an adsorption section and a rectification section, and heat and/or a stripping gas is fed into the column bottom.

10. The process according to claim 9, wherein a gas stream which comprises propene and is obtained in the desorption step E) is fed into the column bottom of the absorption column as stripping gas.

11. The process according to any of claims 1 to 10, wherein stripping is effected in step F) with steam.

12. The process according to claim 11, wherein steam is condensed out of and removed as water, or steam is removed by adsorption, rectification and/or membrane separation, from the gas stream f1 which comprises propene and steam and is obtained in step F) by one- or multistage cooling and compression.

13. The process according to any of claims 1 to 12, wherein the offgas stream d2 which comprises propane and is obtained in step D) is recycled at least partly into the dehydrogenation zone.

14. The process according to any of claims 1 to 13, wherein at least a portion of the gas stream d2 which comprises propane and is obtained in step D) is contacted in a further step G) with a high-boiling absorbent and the gases dissolved in the absorbent are subsequently desorbed to obtain a recycle stream g1 consisting substantially of propane and an offgas stream g2 comprising methane, ethane, ethene, carbon monoxide, carbon dioxide and hydrogen, and the recycled stream g1 consisting substantially of propane is recycled into the dehydrogenation zone.

15. The process according to claim 14, wherein the high-boiling absorbent used in step G) is selected from the group consisting of C₄-C₁₈-alkanes, naphtha and the middle oil fraction from paraffin distillation.

16. The process according to claim 14 or 15, wherein the gases dissolved in the absorbent are desorbed in step G) by stripping with stream.

17. The process according to any of claims 1 to 13, wherein carbon dioxide is removed by gas scrubbing in a further step G) at least from a substream of the gas stream d2 which comprises propane and is obtained in step D), to obtain a low-carbon dioxide recycle stream g1 which is recycled into the dehydrogenation zone.

## Revendications

1. Procédé de fabrication de propène à partir de propane selon les étapes
A) un courant gazeux d'alimentation a contenant du propane est mis à disposition ;
B) le courant gazeux d'alimentation a contenant du propane, éventuellement de la vapeur d'eau et éventuellement un courant gazeux contenant de l'oxygène sont introduits dans une zone de déshydrogénation et le propane est soumis à une déshydrogénation en propène, un courant gazeux de produits b contenant du propane, du propène, du méthane, de l'éthane, de l'éthène, du monoxyde de carbone, du dioxyde de carbone, de la vapeur d'eau, éventuellement de l'hydrogène et éventuellement de l'oxygène étant obtenu ;
C) le courant gazeux de produits b est refroidi, éventuellement comprimé et la vapeur d'eau est éliminée par condensation, un courant gazeux de produits c appauvri en vapeur d'eau étant obtenu ;
D) le courant gazeux de produits c) est mis en contact dans une première zone d'absorption avec un agent d'absorption inerte à action sélective, qui absorbe sélectivement le propène, un courant d'agent d'absorption d1 chargé essentiellement avec du propène et un courant gazeux d2 contenant du propane, du propène, du méthane, de l'éthane, de l'éthène, du monoxyde de carbone, du dioxyde de carbone, éventuellement de l'hydrogène et éventuellement de l'oxygène étant obtenus ;
E) le courant d'agent d'absorption d1 est éventuellement détendu à une pression inférieure dans une première zone de désorption, un courant d'agent d'absorption e1 chargé essentiellement avec du propène et un courant gazeux e2 contenant du propène étant obtenus, le courant gazeux e2 étant recyclé dans la première zone d'absorption,
F) à partir du courant d'agent d'absorption d1 ou e1 chargé essentiellement avec du propène, dans au moins une seconde zone de désorption, un courant gazeux f1 contenant du propène est libéré par détente, chauffage et/ou extraction du courant d'agent d'absorption d1 ou e1, l'agent d'absorption à action sélective étant récupéré.

2. Procédé selon la revendication 1, **caractérisé en ce que** la déshydrogénation à l'étape B) est réalisée sous la forme d'une déshydrogénation oxydative ou non oxydative.

3. Procédé selon la revendication 1, **caractérisé en ce que** la déshydrogénation à l'étape B) est réalisée sous forme adiabatique ou isotherme.

4. Procédé selon la revendication 1, **caractérisé en ce que** la déshydrogénation à l'étape B) est réalisée dans un réacteur à lit solide, un réacteur à lit mobile ou un réacteur à lit fluidisé.

5. Procédé selon la revendication 1, **caractérisé en ce qu'**un courant gazeux contenant de l'oxygène est introduit à l'étape B), le courant gazeux contenant de l'oxygène contenant au moins 90 % en volume d'oxygène.

6. Procédé selon la revendication 5, **caractérisé en ce que** la déshydrogénation est réalisée sous la forme d'une déshydrogénation autotherme.

7. Procédé selon l'une quelconque des revendications 1 à 6, **caractérisé en ce qu'**une partie du courant gazeux f1 contenant du propène obtenu à l'étape F) est recyclée dans la zone d'absorption.

8. Procédé selon l'une quelconque des revendications 1 à 7, **caractérisé en ce que** l'agent d'absorption à action sélective utilisé à l'étape D) est choisi dans le groupe constitué par la NMP, les mélanges NMP/eau contenant jusqu'à 20 % en poids d'eau, le m-crésol, l'acide acétique, la méthylpyrazine, le dibromométhane, le DMF, le carbonate de propylène, la N-formylmorpholine, le carbonate d'éthylène, le formamide, le malonodinitrile, la gamma-butyrolactone, le nitrobenzène, le DMSO, le sulfolane, le pyrrol, l'acide lactique, l'acide acrylique, l'acide 2-chloropropionique, le triméllitate de triallyle, le triméllitate de tris(2-éthylhexyle), le phtalate de diméthyle, l'ester diméthylique de l'acide succinique, l'acide 3-chloropropionique, la morpholine, l'acétonitrile, le sulfate de 1-butyl-3-méthylimidazoliniumoctyle, le tosylate d'éthylméthylimidazolinium, l'adiponitrile, la diméthylaniline et l'acide formique.

9. Procédé selon l'une quelconque des revendications 1 à 8, **caractérisé en ce que** la zone d'absorption de l'étape D) est configurée sous la forme d'une colonne d'absorption munie d'une partie d'absorption et d'une partie de rectification, et de la chaleur et/ou un gaz d'extraction est introduit dans le fond de la colonne.

10. Procédé selon la revendication 9, **caractérisé en ce qu'**un courant gazeux contenant du propène, qui est obtenu dans l'étape de désorption E), est introduit dans le fond de la colonne d'absorption en tant que gaz d'extraction.

11. Procédé selon l'une quelconque des revendications 1 à 10, **caractérisé en ce que** l'extraction de l'étape F) est réalisée avec de la vapeur d'eau.

12. Procédé selon la revendication 11, **caractérisé en ce qu'**à partir du courant gazeux f1 contenant du propène et de la vapeur d'eau obtenu à l'étape F), de la vapeur d'eau est condensée et séparée sous forme d'eau par refroidissement et compression en une ou plusieurs étapes ou de la vapeur d'eau est séparée par adsorption, rectification et/ou séparation sur membrane.

13. Procédé selon l'une quelconque des revendications 1 à 12, **caractérisé en ce que** le courant de gaz d'échappement d2 contenant du propane obtenu à l'étape D) est recyclé au moins partiellement dans la zone de déshydrogénation.

14. Procédé selon l'une quelconque des revendications 1 à 13, **caractérisé en ce qu'**au moins une partie du courant gazeux d2 contenant du propane obtenu à l'étape D) est mise en contact lors d'une étape supplémentaire G) avec un agent d'absorption à point d'ébullition élevé et les gaz dissous dans l'agent d'absorption sont ensuite désorbés, un courant de recyclage g1 essentiellement constitué de propane et un courant de gaz d'échappement g2 contenant du méthane, de l'éthane, de l'éthène, du monoxyde de carbone, du dioxyde de carbone et de l'hydrogène étant obtenus, et le courant de recyclage g1 essentiellement constitué de propane est recyclé dans la zone de déshydrogénation.

15. Procédé selon la revendication 14, **caractérisé en ce que** l'agent d'absorption à point d'ébullition élevé utilisé à l'étape G) est choisi dans le groupe constitué par les alcanes en C₄-C₁₈, le naphta et la fraction d'huile moyenne issue de la distillation de la paraffine.

16. Procédé selon la revendication 14 ou 15, **caractérisé en ce que** l'extraction destinée à la désorption des gaz dissous dans l'agent d'absorption à l'étape G) est réalisée avec de la vapeur d'eau.

17. Procédé selon l'une quelconque des revendications 1 à 13, **caractérisé en ce qu'**à partir d'au moins une partie du courant gazeux d2 contenant du propane obtenu à l'étape D), du dioxyde de carbone est séparé lors d'une étape supplémentaire G) par épuration gazeuse, un courant de recyclage g1 pauvre en dioxyde de carbone étant obtenu, qui est recyclé dans la zone de déshydrogénation.
